# EUROPEAN PATENT SPECIFICATION

(11) **EP 1 517 677 B1**
(45) Date of publication and mention of the grant of the patent: **10.03.2010**
(21) Application number: 03730458.1
(22) Date of filing: 18.06.2003
(51) Int. Cl.: A61K 9/20, A61K 47/26, A61K 47/08, A61K 47/16

(54) **POPPING ORAL ADMINISTRATION FORM**
PUFFENDE ORALE DARREICHUNGSFORMEN
FORME PHARMACEUTIQUE DESTINEE A UNE ADMINISTRATION PAR VOIE ORALE, QUI ECLATE DANS LA BOUCHE

(30) Priority: 19.06.2002 US 173814
(43) Date of publication of application: 30.03.2005
(73) Proprietor: CTS Chemical Industries Ltd., Kiryat Malachi 70953 (IL)
(72) Inventor: FIRST, Sigal, 47224 Ramat HaSharon (IL); YAMIN, Rina, 76516 Rehovot (IL)
(74) Representative: Hiebl, Inge Elisabeth
(86) International application number: PCT/IL2003/000519
(87) International publication number: WO 2004/000283

(56) References cited:
- EP-A- 0 371 228
- WO-A-02/062152
- US-A- 4 263 328
- US-A- 4 271 206
- US-A- 4 289 794
- US-A- 6 150 424

## Description

### FIELD OF THE INVENTION

This invention relates to oral administration forms and to processes for their preparation.

### BACKGROUND OF THE INVENTION

Gasified particles are known in the art as particles that comprise a core material, which encapsulates a pressurized gas that escapes as the core material dissolves or shatters.

Processes for making gasified particles are known in the art of candy making. For example, processes for making gasified confections are described in the following publications: U.S. Patent Nos. 3,985,909, 3,985,910 and 4,001,457.

U.S. Patent No. 4,289,794 describes a method for preparing gasified candy whereby a sugar melt is gasified at superatmospheric pressure and then is cooled below its fusion temperature under superatmospheric pressure to form a gasified candy. As the gasified candy is wetted in the mouth the candy melts and the gas escapes producing an entertaining popping sensation.

WO 99/64555 describes laundry detergent products that include gasified particles, and may also include other materials such as bleaching agents and conventional detergent composition adjuvants. The gasified particles that are used in the laundry detergent are said to increase the rate of product dissolution in the wash water and add desirable product aesthetics in the form of colored speckles. In addition, the gasified particles can provide both audible and olfactory signals to the consumer that the product is working.

Another use of gasified particles is described in U.S. Patent No. 6,310,014 wherein a composition comprising a gasified solid and an anhydrous liquid base is described. Such composition is said to be useful for personal and household care and to deliver an audible cracking or popping sound during used.

Gasified particles are also known for use in cosmetic treatments, particularly hair treatments as described in EP 1059076. The cosmetic compositions contain gasified particles and the gas is released on contact with water or moisture.

Effervescent pharmaceutical compositions for oral administration are also known in the art. An effervescent pharmaceutical composition includes compounds which evolve gas by means of a chemical reaction which takes place upon exposure of the effervescent pharmaceutical composition to water or other fluids.

Pharmaceutical compositions comprising an effervescent agent are described for example, in US Patent Nos. 6,350,470 and 5,178,878 and in EP 1082106.

US-A-4,271,206 relates to uniformly shaped pieces of gasified candy containing gas bubbles having a diameter below 150 microns producing a prolonged sizzling sensation when permitted to dissolve in the mouth.

EP 0 371 228 relates to hard candy containing an enzyme in which pressurized gas is entrapped in the form of fine bubbles to issue pleasant sounds, as it dissolves in the mouth, so that a consumer can intake the enzyme with a certain pleasure and without any feel of drug dosage.

US-A-4,263,328 relates to a confectionary composition, in particular a gasified candy, which, when placed in the mouth, produces an entertaining but short-lived popping sensation.

WO 02/062152 relates to a water-soluble or water-dispersible tablet based on a carbohydrate matrix containing entrapped gas and sufficient closed porosities to allow retention of the entrapped gas in an mount which promotes dissolution or dispersion of the tablet on contact with water.

### SUMMARY OF THE INVENTION

The present invention provides, according to the first aspect thereof, an oral pharmaceutical composition comprising an active ingredient selected from a vitamin, a mineral, an expectorant, an analgesic, an antipyretic, an anti-inflammatory, an antibiotic, an anti-hypertensive or an anti-histamine agent or combinations thereof and pressurized gas, said pressurized gas being trapped in cavities within a pharmaceutically acceptable material, in a manner that allows the gas to escape upon dissolution or shattering of said pharmaceutically acceptable material for use in a method of oral administration of the active ingredient to a subject in need thereof for the purpose of achieving a therapeutic effect and stimulating saliva production, wherein said subject belongs to the pediatric or the elderly population. An administration form/pharmaceutical composition according to the invention may comprise more than one active ingredient, and it preferably comprises a pharmaceutically acceptable carrier.

The term "pressurized gas" refers to a gas at a pressure more than 1 atmosphere.

The oral administration form of the present invention may further comprise coloring, flavoring and other pharmaceutical or neutraceutical excipients.

It should be noted that the term *"administration form"* should be construed in a broad sense and includes any form administered for the purpose of achieving a therapeutic effect in humans or animals. It may be sold as a pharmaceutical administration form carrying a label as to the intended indication, whether as a prescription drug or over the counter, or it may be sold without any specific indication, for example as a neutraceutical (neutraceuticals are often referred to as *"food additives" or "food supplements"*).

*"An oral administration form"* is an administration form, the swallowing of which is permissible. Such an administration form is usually intended to be given through the mouth, for swallowing, for treating the mouth cavity, etc.

The term *"active ingredient"* should be construed in a broad sense as including any ingredient considered to have a therapeutic effect when delivered to a subject in need thereof. The active ingredient is selected from an analgesic, an "antipyretic agent, an anti-inflammatory agent, a vitamin, an expectorant, an antibiotic, an anti-hypertensive, an anti-histamine, a mineral or combinations thereof. Thus, it may be a drug such as paracetamol, diphenydramine, dextromethorphan, lidocaine, loratadine, ibuprofen, pseudoephedrine, enalapril, calcium carbonate, etc, a vitamin or mineral such as vitamin C, vitamin E, biotin, selenium, zinc, etc., a food additive such as Echinacea, propolis, soy extract, etc. or a veterinary active ingredient such as nitroscanate, abamectin, ivermectin, etc. The active ingredient may be taste masked, for instance by coating or microencapsulation.

Non limiting examples of suitable materials for trapping therein the gas are sugars such as glucose, fructose, sucrose, lactose, maltose, corn syrup and mixtures thereof.

The gas trapped in the cavities may be any pharmaceutically acceptable inert gas. The term "*inert*" indicates that the gas does not react with the pharmaceutically acceptable material, in the cavities of which the pressurized gas is trapped and the other ingredients included in the administration form during preparation, storage or use. Non limiting examples of gases suitable for the preparation of the oral administration form are carbon dioxide, nitrogen, air, helium, argon, and neon.

An oral administration form according to the present invention may have benefits in many circumstances, for instance, it may be popular with children that will like the popping sensation and will be more willing to take a popping administration form than one that does not create a popping sensation. The escape of the gas does not only produce a pleasant sensation but may also stimulate saliva production, thereby providing additional saliva to aid dissolution in the mouth. Similarly, it may be used to enhance dissolution of tablets or powders in a drinking liquid. Such tablets may be useful for the elderly or swallow-problem population. It may be used in semi-solids, oils, suspensions or solid preparations to enhance disintegration or dissolution of the active ingredients either in the mouth or in the stomach or intestine.

Particles which contain trapped pressurized gas may be coated by any suitable material that would protect them from direct contact with water or moisture during storage. However, such a coating material should dissolve when the escape of the gas is required. Suitable coating materials may be for instance, cocoa butter that melts in the mouth, biodegradable polymers typically used for gastrointestinal delivery of drugs (such as enteric polymer that dissolves in the intestine), etc.

The oral administration form according to the present invention may have different forms, such as a tablet, powder, pellets, capsule, syrup, oil, suspension, gel, drops and various candy-like forms.

In one non-limiting example, candy-like administration form may be a chocolate bar including gasified particles and an active ingredient.

The present invention further provides a method for preparing a gasified oral administration form according to the present invention, the method comprising:
i) preparing a mixture comprising (a) an active ingredient, and (b) a pharmaceutically acceptable material trapping pressurized gas within cavities thereof; and
ii) processing the mixture to obtain an administration form, said processing being under conditions that permits said gas to escape upon dissolution or shattering of the administration form.

The mixture mentioned in (i) above may comprise more than one active ingredient.

According to one embodiment, the mixture prepared in (i) also comprises a pharmaceutically acceptable ingredient (c) that melts in the mouth, such as cocoa butter. According to this embodiment, the mixture obtained in (i) includes the ingredients (a) and (b) of the mixture, homogeneously dispersed in ingredient (c), and the processing mentioned in (ii) includes casting of the mixture into molds and cooling obtain the gasified oral administration form.

According to another embodiment, the mixture prepared in (i) is of powders, and the processing mentioned in (ii) includes compressing the mixture to produce a tablet.

The present invention further provides a method for preparing a gasified oral administration form comprising:
i) melting a pharmaceutically acceptable material to obtain a melt;
ii) adding a gas and at least one active ingredient and optionally a pharmaceutically acceptable excipient into the melt under superatmospheric pressure to obtain a liquid pharmaceutical composition;
iii) casting the liquid pharmaceutical composition into a mold under superatmospheric pressure;
iv) solidifying the cast pharmaceutical composition under conditions suitable to obtain a gasified oral administration form; and releasing the pressure.

The solidification mentioned above in (iv) may be done in a mold having the form of a desired administration form, for instance, a tablet.

Alternatively, the gasified pharmaceutical composition obtained by the above method in (iii) may be processed to an oral administration form according to the present invention by the following steps:
(iv) solidifying said gasified pharmaceutical composition to obtain a solid gasified pharmaceutical composition;
(v) grinding the solid gasified pharmaceutical composition to obtain a popping powder;
(vi) optionally adding excipients to said powder and mixing them together; and
(vii) processing the obtained powder or mixture to obtain an oral administration form which produces popping sensation when it is wetted.

The active ingredient used in any of the above methods may be coated or microencapsulated with a taste-masking material, enteric polymers, humidity protective materials, oxidation protective materials etc.

According to an additional aspect of the invention there is described a method for orally administering an active ingredient to a patient. The method comprising orally administering to the subject an oral administration form according to the present invention. This method may be used for oral administration of drugs to subjects reluctant to take pharmaceutical compositions that do not create a popping sensation upon wetting.

Further described is a method for treating a patient by orally administering to him an administration form according to the present invention.

### DETAILED DESCRIPTION OF THE INVENTION

In order to understand the invention and to see how it may be carried out in practice, several specific embodiments will now be described, by way of non-limiting examples only.

### Example 1 - Preparation of Paracetamol tablets 500mg

The following ingredients were used in the preparation of the above-mentioned tablets:

| Ingredient mg/tab |
|---|
| Coated Paracetamol 540.0 mg |
| Popping Candy 300.0 mg |
| Cross Povidone 30.0 mg |
| Magnesium Stearate 15.0 mg |
| Aspartame 10.0 mg |
| Flavor 10.0 mg |

Process for the preparation: all the above-mentioned ingredients were mixed together to obtain a uniform mixture that was compressed conventionally to obtain a tablet.

### Example 2 - Preparation of Calcium carbonate lozenges 300mg

The following ingredients were used in the preparation of the above-mentioned lozenges:

| Ingredient mg/loz. |
|---|
| Calcium Carbonate 300.0 mg |
| Sucrose 100.0 mg |
| Lactose 100.0 mg |
| Corn Syrup 50.0 mg |
| FD&C Red # 40 0.02 mg |
| Carbon Dioxide q.s. |
| Grape Flavor 20.0 mg |

Process for the preparation: sucrose, lactose, coloring agent and corn syrup were melted. Calcium carbonate and flavor were added and mixed together with the molten mixture. Carbon dioxide was bubbled into the molten mixture under superatmospheric pressure. Then, still under superatmospheric pressure, the melt mass was cast to lozenges-shaped molds and cooled.

### Example 3 - Preparation of Pseudoephedrine hydrochloride powder for reconstitution

The following ingredients were used in the preparation of the above-mentioned powder:

| Ingredient mg/g |
|---|
| Coated beads of pseudoephedrine hydrochloride 200.0 mg |
| Fructose 275.0 mg |
| Lactose 275.0 mg |
| Liquid Glucose 225.0 mg |
| Nitrogen q.s. |
| Cherry flavor 20.0 mg |
| FD&C Blue # 1 0.002 mg |
| FD&C Red # 40 0.001 mg |
| Saccharin sodium 5.0 mg |

Process for the preparation: fructose, lactose and liquid glucose were melted. Flavor, sweetener and coloring agents were added and mixed together with the molten mixture. Nitrogen was bubbled into the obtained molten mixture under superatmospheric pressure. Then, the molten mixture was cooled under superatmospheric pressure and then the pressure was released. The obtained solid mixture was ground to obtain a popping powder.

Psuedoephedrine beads were mixed together with the obtained popping powder to yield pseudoephedrine hydrochloride powder.

### Example 4- Preparation of Ivermectin 3mg capsules

The following ingredients were used in the preparation of the above-mentioned capsules:

| Ingredient mg/caps. |
|---|
| Ivermectin 3.0 mg |
| Popping candy 100.0 mg |
| Magnesium stearate 1.5 mg |
| Microcrystalline cellulose 150.0 mg |

Process for the preparation: all the above-mentioned ingredients were mixed together to yield a uniform mixture. The obtained mixture was filled inside hard gelatin capsules.

### Example 5 - Preparation of Propolis melt bar

The following ingredients were used in the preparation of the above-mentioned bar:

| Ingredient mg/Bar |
|---|
| Propolis Extract 200.0 mg |
| Popping Candy 1000.0mg |
| Cocoa Butter 800.0 mg |
| Chocolate Flavor 20.0 mg |

Process for the preparation: Cocoa butter was melted and while cooling, propolis extract, chocolate flavor and the popping powder were added. The semi-solid mass was cast inside chocolate bar molds to yield the desired bars.

### Example 6 - Preparation of Lidocaine Gel

The following ingredients were used in the preparation of the above-mentioned gel:

| Ingredient % w/w |
|---|
| Lidocaine base 0.2% |
| Popping Candy (coated with cocoa butter) 20.0% |
| Propylene Glycol 30.0% |
| Carbomer 1.0% |
| Sodium Hydroxide q.s. |
| Water to 100.0% |

Process for the preparation: Carbomer and water are heated and mixed. Sodium Hydroxide is added to form a liquid gel. While cooling, the rest of the materials are added.

### Example 7: Preparation of Ibuprofen 250mg Tablet

| Ingredient mg/tab |
|---|
| Coated Ibuprofen 270mg |
| Hard Fat 500mg |
| Strawberry Flavor 3mg |
| Aspartame 5mg |
| Popping Candy 200mg |

### Process for the preparation

Ibuprofen, Strawberry flavor and Aspartame are mixed in Hard Fat heated to 45°C. The mixture is cooled to 38°C and Popping Candy is added. Mixture is filled inside pre-cooled blisters.

### Example 8: Preparation of Amoxycillin 250mg Tablet

| Ingredient mg/tab |
|---|
| Amoxycillin 250mg |
| Hard Fat 600mg |
| Vanilla Flavor 3mg |
| Aspartame 5mg |
| Popping Candy 250mg |

### Process for the preparation:

Amoxycillin, Vanilla flavor and Aspartame are mixed in Hard Fat heated to 45°C. The mixture is cooled to 38°C and Popping Candy is added. Mixture is filled inside pre-cooled blisters.

## Claims

1. An oral pharmaceutical composition comprising an active ingredient selected from a vitamin, a mineral, an expectorant, an analgesic, an antipyretic, an anti-inflammatory, an antibiotic, an anti-hypertensive or an anti-histamine agent or combinations thereof and pressurized gas, said pressurized gas being trapped in cavities within a pharmaceutically acceptable material, in a manner that allows the gas to escape upon dissolution or shattering of said pharmaceutically acceptable material for use in a method of oral administration of the active ingredient to a subject in need thereof for the purpose of achieving a therapeutic effect and stimulating saliva production, wherein said subject belongs to the pediatric or the elderly population.

2. An oral pharmaceutical composition according to Claim 1, wherein said pharmaceutical acceptable material comprises said active ingredient.

3. An oral pharmaceutical composition according to Claim 1, comprising more than one active ingredient.

4. An oral pharmaceutical composition according to any of the preceding claims wherein said active ingredient includes a prescription drug or a drug sold over the counter.

5. An oral pharmaceutical composition according to any one of the preceding claims, suitable for veterinary use.

6. An oral pharmaceutical composition according to Claim 1, wherein said active ingredient is selected from paracetamol, diphenhydramine, dextromethorphan, loratadine, lidocaine, ibuprofen, propolis extract, pseudoephedrine, amoxycillin, enalapril, vitamin, biotin, selenium, zinc, and calcium carbonate.

7. An oral pharmaceutical composition according to Claim 5, wherein said active ingredient is selected from nitroscanate, abamectin and ivermectin.

8. An oral pharmaceutical composition according to any one of the preceding claims, wherein said active ingredient is coated by a coating.

9. An oral pharmaceutical composition according to Claim 8, wherein said coating comprises taste-masking materials, biodegradable polymers, enteric polymers, humidity protective materials and/or oxidation protective materials.

10. An oral pharmaceutical composition according to Claim 8, wherein said coating is a taste masking coating.

11. An oral pharmaceutical composition according to any one of the preceding claims, having a form of a tablet, powder, pellets, capsule, syrup, oil, suspension, gel, drops, or candy-like form.

12. An oral pharmaceutical composition according to any one of the preceding claims, wherein said pharmaceutically acceptable material is selected from sugars, corn syrup or mixtures thereof.

13. An oral pharmaceutical composition according to any one of the preceding claims, wherein said gas is selected from carbon dioxide, nitrogen, air, helium, argon, and neon.

14. An oral pharmaceutical composition according to any one of the preceding claims that is dissoluted in the mouth.

15. An oral pharmaceutical composition according to one of the preceding claims wherein the pressurized gas is supplied by a popping candy.

## Patentansprüche

1. Orale pharmazeutische Zusammensetzung, umfassend ein aktives Ingrediens, ausgewählt aus einem Vitamin, einem Mineral, einem Expektorans, einem Analgetikum, einem Antipyretikum, einem anti-inflammatorischen Mittel, einem Antibiotikum, einem Antihypertensivum und einem Anti-Histaminikum und Kombinationen davon, und Druckgas, wobei das Druckgas in Hohlräumen in einem pharmazeutisch verträglichen Material derart eingeschlossen ist, dass das Gas bei Auflösen oder Zerfallen des pharmazeutisch verträglichen Materials entweichen kann, zur Verwendung in einem Verfahren zur oralen Verabreichung des aktiven Ingrediens an ein Subjekt, das Bedarf dafür hat, zum Zweck der Erreichung eines therapeutischen Effekts und der Stimulierung der Speichelproduktion, wobei das Subjekt zu der pädiatrischen oder der älteren Population gehört.

2. Orale pharmazeutische Zusammensetzung gemäß Anspruch 1, wobei das pharmazeutisch verträgliche Material das aktive Ingrediens umfasst.

3. Orale pharmazeutische Zusammensetzung gemäß Anspruch 1, die mehr als ein aktives Ingrediens umfasst.

4. Orale pharmazeutische Zusammensetzung gemäß einem der vorangehenden Ansprüche, wobei das aktive Ingrediens ein Verschreibungsarzneimittel oder ein Arzneimittel, das über die Ladentheke verkauft wird, umfasst.

5. Orale pharmazeutische Zusammensetzung gemäß einem der vorangehenden Ansprüche, die zur veterinären Verwendung geeignet ist.

6. Orale pharmazeutische Zusammensetzung gemäß Anspruch 1, wobei das aktive Ingrediens ausgewählt ist aus Paracetamol, Diphenhydramin, Dextromethorphan, Loratadin, Lidocain, Ibuprofen, Propolis-Extrakt, Pseudoephedrin, Amoxycillin, Enalapril, Vitamin, Biotin, Selen, Zink und Calciumcarbonat.

7. Orale pharmazeutische Zusammensetzung gemäß Anspruch 5, wobei das aktive Ingrediens aus Nitroscanat, Abamectin und Ivermectin ausgewählt ist.

8. Orale pharmazeutische Zusammensetzung gemäß einem der vorangehenden Ansprüche, wobei das aktive Ingrediens mit einem Überzug überzogen ist.

9. Orale pharmazeutische Zusammensetzung gemäß Anspruch 8, wobei der Überzug geschmacksmaskierende Materialien, biologische abbaubare Polymere, magensaftresistente Polymere, vor Feuchtigkeit schützende Materialien und/oder vor Oxidation schützende Materialien umfasst.

10. Orale pharmazeutische Zusammensetzung gemäß Anspruch 8, wobei der Überzug ein geschmacksmaskierender Überzug ist.

11. Orale pharmazeutische Zusammensetzung gemäß einem der vorangehenden Ansprüche, die die Form einer Tablette, eines Pulvers, von Pellets, einer Kapsel, eines Sirups, eines Öls, einer Suspension, eines Gels, von Tropfen oder eine Süßigkeit-artige Form hat.

12. Orale pharmazeutische Zusammensetzung gemäß einem der vorangehenden Ansprüche, wobei das pharmazeutisch verträgliche Material aus Zuckern, Maissirup und Gemischen davon ausgewählt ist.

13. Orale pharmazeutische Zusammensetzung gemäß einem der vorangehenden Ansprüche, wobei das Gas aus Kohlendioxid, Stickstoff, Luft, Helium, Argon und Neon ausgewählt ist.

14. Orale pharmazeutische Zusammensetzung gemäß einem der vorangehenden Ansprüche, die im Mund gelöst wird.

15. Orale pharmazeutische Zusammensetzung gemäß einem der vorangehenden Ansprüche, wobei das Druckgas durch eine puffende Süßigkeit zugeführt wird.

## Revendications

1. Composition pharmaceutique orale comprenant un ingrédient actif choisi parmi une vitamine, un minéral, un expectorant, un analgésique, un antipyrétique, un anti-inflammatoire, un antibiotique, un antihypertenseur ou un antihistaminique ou des combinaisons de ceux-ci et du gaz comprimé, dans laquelle le gaz comprimé est inclu dans des cavités à l'intérieur d'un matériau pharmaceutiquement acceptable de manière permettant la libération du gaz à la dissolution ou à la désintégration dudit matériau pharmaceutiquement acceptable pour l'utilisation dans un procédé d'administration par voie orale de l'ingrédient actif à un sujet ayant besoin de celui-ci pour obtenir un effet thérapeutique et stimuler la production de la salive, le sujet faisant partie de la population pédiatrique ou de la population des personnes âgées.

2. Composition pharmaceutique orale selon la revendication 1, dans laquelle le matériau pharmaceutiquement acceptable comprend ledit ingrédient actif.

3. Composition pharmaceutique orale selon la revendication 1 comprenant plus d'un ingrédient actif.

4. Composition pharmaceutique orale selon l'une quelconque des revendications précédentes, dans laquelle l'ingrédient actif comprend un médicament d'ordonnance ou un médicament sans ordonnance.

5. Composition pharmaceutique orale selon l'une quelconque des revendications précédentes appropriée pour l'utilisation vétérinaire.

6. Composition pharmaceutique orale selon la revendication 1, dans laquelle l'ingrédient actif est choisi parmi le paracétamol, le diphénhydramine, le dextrométhorphane, le loratadine, le lidocaine, l'ibuprofène, l'extrait de propolis, le pseudoéphédrine, l'amoxycilline, l'enalapril, une vitamine, la biotine, le sélénium, le zinc et le carbonate de calcium.

7. Composition pharmaceutique orale selon la revendication 5, dans laquelle l'ingrédient actif est choisi parmi le nitroscanate, l'abamectine et l'ivermectine.

8. Composition pharmaceutique orale selon l'une quelconque des revendications précédentes, dans laquelle l'ingrédient actif est enrobé d'un enrobage.

9. Composition pharmaceutique orale selon la revendication 8, dans laquelle l'enrobage comprend des matériaux masquant le goût, des polymères biodégradables, des polymères entériques, des matériaux protecteurs contre l'humidité et/ou des matériaux protecteurs contre l'oxydation.

10. Composition pharmaceutique orale selon la revendication 8, dans laquelle l'enrobage est un enrobage masquant le goût.

11. Composition pharmaceutique orale selon l'une quelconque des revendications précédentes sous la forme d'un comprimé, d'une poudre, des pellets, d'une capsule, d'un sirop, d'une huile, d'une suspension, d'un gel, des gouttes ou sous la forme de sucrerie.

12. Composition pharmaceutique orale selon l'une quelconque des revendications précédentes, dans laquelle le matériau pharmaceutiquement acceptable est choisi parmi les sucres, le sirop de maïs et les combinaisons de ceux-ci.

13. Composition pharmaceutique orale selon l'une quelconque des revendications précédentes, dans laquelle ledit gaz est choisi parmi le dioxyde de carbone, le nitrogène, l'air, l'hélium, l'argon et le néon.

14. Composition pharmaceutique orale selon l'une quelconque des revendications précédentes qui est dissoue en bouche.

15. Composition pharmaceutique orale selon l'une quelconque des revendications précédentes, dans laquelle le gaz comprimé est fourni par un «popping candy».
